Europäisches Patentamt

European Patent Office

Office européen des brevets

(19)

(11) Numéro de publication : **0 310 476 B1**

(12)

# FASCICULE DE BREVET EUROPEEN

(45) Date de publication du fascicule du brevet :
**04.03.92 Bulletin 92/10**

(51) Int. Cl.[5] : **A61K 33/16,** A61K 7/48,
A01N 59/10, A61K 33/14

(21) Numéro de dépôt : **88402358.1**

(22) Date de dépôt : **19.09.88**

(54) **Composition inhibitrice ou destructrice d'au moins un être vivant unicellulaire renfermant du fluor F- et du lithium Li+.**

(30) Priorité : **22.09.87 FR 8713086**

(43) Date de publication de la demande :
**05.04.89 Bulletin 89/14**

(45) Mention de la délivrance du brevet :
**04.03.92 Bulletin 92/10**

(84) Etats contractants désignés :
**AT BE CH DE ES FR GB GR IT LI LU NL SE**

(56) Documents cités :
**EP-A- 0 055 109
EP-A- 0 162 574
EP-A- 0 226 187
WO-A-87/07143
US-A- 4 097 590
US-A- 4 473 547
CHEMICAL ABSTRACTS, vol. 105, no. 25, 22
décembre 1986, page 20, résumé no. 218343x,
Columbus, Ohio, US; M. IIDA et al.: "Effects of
fluorides on oral microorganisms", & STUD.
ENVIRON. SCI. 1986, 27(FLUORIDE RES.
1985), 267-76**

(73) Titulaire : **ATLANTIC PHARMACEUTICAL
PRODUCTS LIMITED
Celbridge
Dunaghcumper Co Kildare (IE)**

(72) Inventeur : **Bourbon, Pierre
36, rue Volta
F-31000 Toulouse (FR)**
Inventeur : **Lagny, Pierre
Castle Town Court 6
Celbridge c/o Kildare (IR)**
Inventeur : **Billot, Pierre
20bd. de la Saussaye
F-92200 Neuilly/Seine (FR)**

(74) Mandataire : **Derambure, Christian
Cabinet Bouju Derambure (Bugnion) S.A. 55,
rue Boissonade
F-75014 Paris (FR)**

Jouve, 18, rue Saint-Denis, 75001 PARIS

## Description

L'invention concerne l'inhibition ou destruction des êtres vivants unicellulaires tels que protozoaires, microbes, bactéries, gamètes, champignons, levures ou autres et des virus. Elle vise donc en particulier les domaines techniques de la contraception locale, de l'antibiothérapie, de l'antisepsie, que ce soit dans le cadre de la pharmacie ou de la cosmétique, ainsi que de la désinfection.

On connaît déjà de nombreuses substances et compositions inhibant ou détruisant les êtres vivants unicellulaires. La demande de brevet européen EP-A-0 253 037 mentionne de telles substances et compositions et enseigne déjà qu'il est avantageux d'utiliser un composé libérant l'ion F⁻ seul ou en combinaison avec un autre principe actif de base.

Par ailleurs, on connaît déjà le lithium à l'état ionique dans diverses applications thérapeutiques : en neurologie à titre de normothymique, en rhumatologie ou urologie à titre d'urico-éliminateur, dans le domaine des soins dentaires à titre d'antiseptique (PYOREX, marque déposée), et dans le traitement des affections des voies respiratoires en pneumologie (THIOPHEOL).

On connaît aussi déjà le fluorure de lithium en tant que composé chimique (The MERCK INDEX, TENTH EDITION, page 793, référence 5357). Ce composé n'est cependant pas utilisé à titre de médicamment, ni pour inhiber ou détruire les êtres vivants unicellulaires.

La demande de brevet européen 0 055 109 décrit une composition anti-caries comprenant un sel de fluor et un hydrate de carbone. Parmi les fluorures utilisés à titre d'agent anti-caries on mentionne le fluorure de lithium. Mais ce document ne met en évidence aucune propriété particulière dûe au fluorure de lithium pour inhiber ou détruire les micro-organismes. En effet, dans la lutte contre la carie dentaire, le fluor a une action connue qui ne consiste pas à inhiber ou détruire les micro-organismes.

La demande de brevet européen 0 162 574 décrit une composition d'hygiène bucco-dentaire telle qu'un dentifrice comprenant un sel de fluor, un sel de zinc, un agent tampon et un excipient. Parmi les sels de fluor cités, on mentionne le fluorure de lithium. Cependant les sels de fluor ne sont utilisés dans le cadre de ce document qu'à titre d'anti-caries comme cela est enseigné dans la demande de brevet européen 0 055 109. Par ailleurs, la demande de brevet européen 0 162 574 mentionne également la possibilité d'utiliser un agent tensioactif cationique à titre d'antiseptique et d'antibactérien. Mais ce document ne décrit pas une composition permettant d'inhiber ou de détruire des micro-organismes comportant du fluorure de lithium.

L'invention a pour objets de remédier aux inconvénients connus de l'état de la technique, à savoir :
– de réduire les concentrations nécessaires en principes actifs et/ou principes activateurs en conservant la même efficacité, afin de limiter ou d'éviter les effets secondaires,
– de proposer une composition inhibitrice ou destructrice d'êtres vivants unicellulaires et administrable par voie générale ou parentérale sans danger,
– de fournir un nouveau composé utilisable à titre de médicament ou de produit bactéricide, antibiotique, virucide, antiseptique, désinfectant ou de contraceptif,
– de proposer d'autres applications thérapeutiques du lithium ionique,
– de fournir une composition active contre certains êtres pathogènes contre lesquels on ne connaît pas de remède.

L'invention concerne donc une composition spermicide caractérisée en ce qu'elle renferme du lithium ionique ou ionisable, un principe spermicide, notamment un tensioactif surfactif tel qu'un ammonium quaternaire comme le chlorure de benzalkonium, ou un nonoxynol, et un excipient approprié.

L'invention concerne aussi une composition spermicide caractérisée en ce qu'elle renferme A) du fluorure de lithium, B) un spermicide, notamment un tensioactif surfactif tel qu'un ammonium quaternaire comme le chlorure de benzalkonium, ou un nonoxynol, et C) un excipient approprié.

L'invention concerne également une composition spermicide caractérisée en ce qu'elle renferme A) du fluorure de lithium, B) un spermicide, notamment un tensioactif surfactif tel qu'un ammonium quaternaire comme le chlorure de benzalkonium, ou un nonoxynol, et C) un agent conservateur, notamment comportant un mélange de 5-chloro-2-méthyl-4-isothiazoline-3-one et de 2-méthyl-4-isothioazoline-3-one et D) un excipient approprié.

L'invention concerne également une composition pharmaceutique caractérisée en ce qu'elle comporte du fluorure de lithium et un mélange de 5-chloro-2-méthyl-4-isothiazoline-3-one et de 2-méthyl-4-isothiazoline-3-one.

L'invention concerne également une telle composition pharmaceutique caractérisée en ce qu'elle comporte du lithium ionique ou ionisable et un principe antibiotique.

L'invention concerne également une composition destinée à la désinfection de surfaces telles que les sols ou les instruments caractérisée en ce qu'elle renferme du lithium ionique et ionisable, un principe désinfectant et un excipient approprié.

L'invention concerne également une composition destinée à la désinfection de surfaces telles que les sols ou les instruments caractérisée en ce qu'elle renferme A) du fluorure de lithium, B) un tensioactif surfactif tel qu'un ammonium quaternaire comme le chlorure de benzalkonium, ou un nonoxynol, et C) un excipient approprié.

L'invention concerne également une telle composition caractérisée en ce qu'elle comporte en outre un autre sel de fluor, notamment du fluorure de sodium.

L'invention concerne également l'application du cation $Li^+$ lithium pour obtenir une composition destinée à détruire ou inhiber les gamètes -notamment les spermatozoïdes- à titre de contraceptif local.

L'invention concerne également l'application du cation $Li^+$ lithium pour obtenir une composition pharmaceutique destinée à détruire ou inhiber un être vivant unicellulaire ou virus en dehors de la sphère bucco-dentaire.

L'invention concerne également l'application du cation $Li^+$ lithium pour obtenir une composition destinée à lutter contre les maladies sexuellement transmissibles.

L'invention concerne également l'application du cation $Li^+$ lithium pour obtenir une composition antibiotique.

L'invention concerne également l'application du cation $Li^+$ lithium pour obtenir une composition antiseptique pour la désinfection locale du corps humain ou animal en dehors de la sphère bucco-dentaire.

L'invention concerne également l'application d'au moins un composé susceptible de libérer l'ion $F^-$ et l'ion $Li^+$ -notamment du fluorure de lithium- pour obtenir une composition pharmaceutique destinée à détruire ou inhiber un être vivant unicellulaire ou virus en dehors de la sphère bucco-dentaire.

L'invention concerne également l'application d'au moins un composé susceptible de libérer l'ion $F^-$ et l'ion $Li^+$ -notamment du fluorure de lithium- pour obtenir une composition destinée à lutter contre les maladies sexuellement transmissibles.

L'invention concerne également l'application d'au moins un composé susceptible de libérer l'ion $F^-$ et l'ion $Li^+$ -notamment du fluorure de lithium- pour obtenir une composition contraceptive locale.

L'invention concerne également l'application d'au moins un composé susceptible de libérer l'ion $F^-$ et l'ion $Li^+$ -notamment du fluorure de lithium- pour obtenir une composition antibiotique.

L'invention concerne également l'application d'au moins un composé susceptible de libérer l'ion $F^-$ et l'ion $Li^+$ -notamment du fluorure de lithium- pour obtenir une composition antiseptique pour la désinfection locale du corps humain ou animal en dehors de la sphère bucco-dentaire.

L'invention concerne également l'application d'au moins un composé susceptible de libérer l'ion $F^-$, d'au moins un composé susceptible de libérer l'ion $Li^+$ -notamment du fluorure de lithium- et d'au moins un principe actif -notamment un tensioactif surfactif tel qu'un ammonium quaternaire comme le chlorure de benzalkonium, ou un nonoxynol- pour obtenir une composition pharmaceutique destinée à détruire ou inhiber un être vivant unicellulaire ou virus en dehors de la sphère bucco-dentaire.

L'invention concerne également l'application d'au moins un composé susceptible de libérer l'ion $F^-$, d'au moins un composé susceptible de libérer l'ion $Li^+$ -notamment du fluorure de lithium- et d'au moins un principe actif -notamment un tensioactif surfactif tel qu'un ammonium quaternaire comme le chlorure de benzalkonium, ou un nonoxynol- pour obtenir une composition contraceptive locale.

L'invention concerne également l'application d'au moins un composé susceptible de libérer l'ion $F^-$, d'au moins un composé susceptible de libérer l'ion $Li^+$ -notamment du fluorure de lithium- et d'au moins un principe actif -notamment un tensioactif surfactif tel qu'un ammonium quaternaire comme le chlorure de benzalkonium, ou un nonoxynol- pour obtenir une composition antibiotique.

L'invention concerne également l'application d'au moins un composé susceptible de libérer l'ion $F^-$, d'au moins un composé susceptible de libérer l'ion $Li^+$ -notamment du fluorure de lithium- et d'au moins un principe actif -notamment un tensioactif surfactif tel qu'un ammonium quaternaire comme le chlorure de benzalkonium, ou un nonoxynol- pour obtenir une composition destinée à lutter contre les maladies sexuellement transmissibles.

L'invention concerne également l'application d'au moins un composé susceptible de libérer l'ion $F^-$, d'au moins un composé susceptible de libérer l'ion $Li^+$ -notamment du fluorure de lithium- et d'au moins un principe actif -notamment un tensioactif surfactif tel qu'un ammonium quaternaire comme le chlorure de benzalkonium, ou un nonoxynol- pour obtenir une composition antiseptique pour la désinfection locale du corps humain ou animal en dehors de la sphère bucco-dentaire.

L'invention concerne également l'application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition pharmaceutique destinée à détruire ou inhiber un virus ou un être vivant unicellulaire.

L'invention concerne également l'application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition contraceptive locale, ou une composition antibiotique, ou une composition destinée à lutter contre les maladies sexuellement transmissibles.

L'invention concerne également l'application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition antiseptique pour la désinfection locale du corps humain ou animal.

L'invention concerne également l'application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, D) d'un excipient approprié, et E) d'un autre sel de fluor, notamment du fluorure de sodium, pour obtenir une composition pharmaceutique destinée à détruire ou inhiber un virus ou un être vivant unicellulaire, ou une composition contraceptive locale, ou une composition antibiotique, ou une composition destinée à lutter contre les maladies sexuellement transmissibles, ou une composition antiseptique pour la désinfection locale du corps humain ou animal.

L'invention concerne également l'application d'au moins un composé susceptible de libérer l'ion $F^-$, d'au moins un composé susceptible de libérer l'ion $Li^+$ -notamment du fluorure de lithium- et d'au moins un principe antibiotique pour fabriquer un produit antibiotique comportant des concentrations en $F^-$, $Li^+$ et en principe antibiotique suffisamment faibles pour autoriser son administration par voie orale ou parentérale.

L'invention concerne également l'application d'au moins un composé susceptible de libérer l'ion $F^-$, d'au moins un composé susceptible de libérer l'ion $Li^+$ -notamment du fluorure de lithium-, d'au moins un principe virucide -notamment un tensioactif surfactif tel qu'un ammonium quaternaire comme le chlorure de benzalkonium- pour fabriquer un produit virucide comportant des concentrations en $F^-$, $Li^+$ et en principe virucide de base suffisamment faibles pour autoriser son administration par voie orale ou parentérale.

L'invention concerne également l'application d'au moins un composé susceptible de libérer l'ion $F^-$, d'au moins un composé susceptible de libérer l'ion $Li^+$ -notamment du fluorure de lithium-, et d'au moins un principe bactéricide ou antibiotique ou fongicide, pour fabriquer une composition destinée à lutter contre les micro-organismes pathogènes tels que Chlamydiae, Gardnerella Vaginalis, Bacille de Ducrey, Candida Albicans, Aspergillus, Streptotoccus, Proteus Vulgaris, Haemophilus Influenzae, Pseudomonas Aeruginosa, Escherichia Coli, Staphylococcus, Mycobacterium, Neisseria Gonorrhoaea, Trichomonas, Treponemas, Sarcina Lutea, Bacillus Subtilis, Klebsiella Pneumoniae, Enterococcus.

L'invention met donc en évidence d'une part l'effet synergique surprenant de l'association de l'anion $F^-$ avec le cation $Li^+$ pour détruire ou inhiber les êtres vivants unicellulaires et à titre de médicament, contraceptif, cosmétique, bactéricide, antibiotique, virucide ou désinfectant, d'autre part de nouvelles applications, données à l'ion $Li^+$, et enfin une activité surprenante de l'association de $F^-$, $Li^+$ et d'un principe actif de base permettant de réduire considérablement les concentrations et notamment d'obtenir des produits utilisables par voie orale ou parentérale.

L'invention consiste également à avoir sélectionné le fluorure de lithium parmi les fluorures possibles mentionnés dans l'état de la technique, et à avoir mis en évidence des propriétés inattendues qualitativement et quantitativement du fluorure de lithium.

L'invention est illustrée par les exemples ci-après, la terminologie et les méthodologies utilisées ci-après ont déjà été définies dans la demande de brevet européen déposée le 08.12.1986 EP-A-0253037, et ne seront donc pas intégralement précisées à nouveau.

La solution de chlorure de benzalkonium utilisée dans les essais contenait au minimum 90% en poids de chlorure de benzalkonium C14 de formule: $(C_6H_5\text{-}CH_2\text{-}CH_3NCH_3\text{-}C_{14}H_{29}^+, Cl^-)$ ou chlorure de myristyldiméthyl benzylammonium. On peut aussi utiliser le chlorure de cocodimethylbenzylammonium ou le chlorure d'alkyldimethylbenzylammonium ou un autre chlorure d'ammonium quaternaire. Tous les pourcentages donnés sont des pourcentages en poids.

Le KATHON CG (marque déposée) est un conservateur connu commercialisé par la Société ROHM AND HAAS COMPANY (U.S.A.) et constitué d'un mélange de deux isothiazolines identifiées selon la nomenclature IUPAC comme le 5 - chloro - 2 méthyl - 4 isothiazoline - 3 - one et le 2 - méthyl - 4- isothiazoline - 3 - one.

I - APPLICATION DE L'INVENTION DANS LE DOMAINE DE LA CONTRACEPTION LOCALE :

EXEMPLE 1 :

La demande de brevet européen n° 86 402 716.4 a montré que l'ion F⁻ seul a une CMI (concentration minimale inhibitrice) selon le test spermicide total de SANDERS-CRAMER selon les normes de l'IPPF de 5ppm soit 0,0005 %.

Le même test réalisé avec du fluorure de lithium Fli seul a montré que le fluorure de lithium seul a une CMI de l'ordre de 3ppm, soit 0,0003 %.

Le même test réalisé avec du chlorure du lithium Cl Li seul a montré que le chlorure de lithium seul a une CMI de 20 ppm, soit 0,002%.

On constate donc un effet spermicide de l'ion Li⁺. Mais surtout, la combinaison F⁻, Li⁺ procure un effet synergique inattendu.

Les proportions de fluorure de lithium contenu dans une composition spermicide selon l'invention doivent être telles que le titre de fluorure de lithium qu'elle peut libérer in vivo est supérieur à 2,5mg/l pour être efficace sans atteindre les doses provoquant des réactions secondaires.

Les proportions libérées in vivo dépendent bien entendu de la forme galénique utilisée. Par exemple, les formes galéniques suivantes fournissent de bons résultats : crème, gelée, ovule, tampon, feuille soluble, comprimé, mousse. Par exemple :

| FORME GALENIQUE | FLI (% en poids) |
|---|---|
| CREME | 0,55 |

EXEMPLE 2 :

Par ailleurs, la demande de brevet européen n° 86 402 716. 4 a montré que l'adjonction de 1 millilitre de solution contenant 0,0001 % d'anion F⁻ (par exemple sous forme de fluorure de sodium) à 1 millilitre de composition, contenant du chlorure de benzalkonium comme principe actif de base, dans le cadre du test spermicide total selon les normes de l'IPPF, permet d'abaisser la CMI du chlorure de benzalkonium d'une valeur de 0,006 % (dans le 1 millilitre de départ) à 0,002 % (dans le 1 millilitre de départ).

Le même test réalisé (in vitro) en remplaçant la solution de 1 millilitre à 0,0001% d'anion F⁻ par une solution de 1 millilitre contenant 0,0001 % de fluorure de lithium a permis d'obtenir une CMI du chlorure de benzalkonium de 0,0009% (dans le 1 millitre de départ).

On constate donc encore un effet potentialisateur. Les résultats maxima (in vitro) ont été obtenus avec une solution de fluorure de lithium à 0,0001%.

Les formes galéniques suivante peuvent être utilisées : crème, gelée, ovule, tampon, feuille soluble, comprimé, mousse. Par exemple :

- CREME : chlorure de benzalkonium    0,90%

        Fli    0,55%

excipients : émulgateur, conservateur tel que KATHON (marque déposée), eau purifiée, parfum q.s.p. 100%.
- GELEE : mêmes formulations que la crème
excipients : dérivés solubles de la cellulose, glycérine, conservateur tel que KATHON (marque déposée), eau purifiée, parfum q.s.p. 100%.
- TAMPON : imbibé de la crème définie ci-dessus.

5

```
    - COMPRIMES : chlorure de benzalkonium    0,020g
                  Fli                          0,010g
```

excipients : bicarbonate de sodium, acide citrique, silice colloïdale, cellulose, stéarate de magnésium, lactose q.s.p. 1 comprimé.

- FEUILLE SOLUBLE : mêmes formulations que la crème

excipients : alcool polyvinylique , glycérine, conservateur tel que KATHON (marque déposée), eau purifiée q.s.p. 100%.

```
    - OVULES : chlorure de benzalkonium       0,017g
               Fli                            0,010g
```

excipients : glycérides semi-synthétiques, conservateur tel que KATHON (marque déposée) q.s.p. 1 ovule.

Un produit contraceptif local, notamment spermicide selon l'invention est caractérisé en ce qu'il contient entre 0,20% et 0,75% -notamment de l'ordre de 0,55%- en poids de fluorure de lithium et entre 0,10% et 1,20% de chlorure de benzalkonium.

II- APPLICATION DE L'INVENTION DANS LA LUTTE CONTRE LES GERMES RESPONSABLES D'INFECTIONS TELLES QUE LES MALADIES SEXUELLEMENT TRANSMISSIBLES.

EXEMPLE 3 : LUTTE CONTRE LES CHLAMYDIAE

La méthodologie des tests effectués afin de démontrer l'application de l'invention dans le cadre de la lutte contre les chlamydiae est celle décrite par les Professeurs F. CATALAN, P. SEDNAOUI, A. MILOVANOVIC et Coll., Institut A. FOURNIER, PARIS déjà mentionnée dans la demande de brevet européen EP-A-0 253 037.

Le test a été effectué sur 12 souches de Chlamydia Trachomatis provenant de milieux hospitaliers, notamment de cas d'urêtrites. Tous les essais ont été pratiqués en double et les résultats comparés à ceux d'une souche témoin répertoriée.

Il a tout d'abord été vérifié que le flurorure de lithium à 1mg/l n'a pas d'activité toxique sur les cellules MAC COY. De plus, le seuil d'activité cytotoxique du chlorure de benzalkonium étant de 0,01% sur les cellules MAC COY, le chlorure de benzalkonium n'est pas non plus toxique sur les cellules MAC COY dans les conditions des essais réalisés.

La CMI correspond à la concentration de chlorure de benzalkonium à partir de laquelle le nombre moyen de colonies dans les boîtes de PETRI contenant la substance active est inférieur au dixième du nombre moyen de colonies dans les boîtes de PETRI sans substance active.

La CMI du chlorure de benzalkonium seul a varié sur les 12 souches sauvages testées de 12 à 180 mg/l. La CMI du chlorure de benzalkonium sur la souche témoin répertoriée est de 18mg/l.

La CMI du chlorure de benzalkonium auquel on a ajouté 1mg/ml de fluorure de lithium a varié sur ces mêmes 12 souches testées de 8 à 160 mg/l. La CMI du chlorure de benzalkonium auquel on a ajouté 1mg/ml sur la souche témoin répertoriée est de 13 mg/l.

On constate donc une forte amélioration des résultats avec le fluorure de lithium.

EXEMPLE 4 : LUTTE CONTRE LE GARDNERELLA VAGINALIS

Deux essais ont été réalisés pour illustrer l'application de l'invention dans la lutte contre le Gardnerella Vaginalis.

Dans le premier essai, on a incorporé directement l'échantillon de substance bactéricide dans le milieu de culture spécifique du germe. Une gamme de concentration de la substance bactéricide a été effectuée, de raison géométrique 2, de 400 $\mu$g/ml à 1,56 $\mu$g/ml. La concentration cellulaire utilisée lors de l'ensemencement des boîtes de Pétri a été de $10^{-3}$ par ml. On a pratiqué une incubation de 24 heures à 37° C, puis déterminé, pour chaque souche sauvage testée, la concentration en principe de base détruisant la totalité de germes (voir méthode du Professeur F. CATALAN et collaborateurs, Institut A. FOURNIER, PARIS). L'essai a été conduit sur 32 souches sauvages de Gardnerella Vaginalis en provenance de milieux hospitaliers, et avec le chlorure de benzalkonium puis le nonoxynol 9 comme principes actifs, et avec le fluorure de sodium FNa à 1 mg/l puis le fluorure de lithium Fli à 1 mg/l comme principes activateurs. Les résultats obtenus sont rassemblés sur le tableau suivant exprimant le nombre de souches inhibées sur les 32 testées selon les concentrations de principe actif de base (chlorure de benzalkonium ou nonoxynol).

| SUBSTANCE | Concentrations en mg/l de principe actif de base (chlorure de benzalkonium ou nonoxynol) | | | | | | | | |
|---|---|---|---|---|---|---|---|---|---|
| | 1,56 | 3,125 | 6,25 | 12,5 | 25 | 50 | 100 | 200 | 400 |
| Chlorure de benzalkonium | 0 | 0 | 1 | 28 | 31 | 32 | – | – | – |
| Chlorure de benzalkonium + FNa (1 mg/l) | 0 | 0 | 7 | 30 | 32 | – | – | – | – |
| Chlorure de benzalkonium + FLi (1 mg/l) | 0 | 0 | 13 | 32 | – | – | – | – | – |
| Nonoxynol 9 | 0 | 0 | 0 | 0 | 4 | 6 | 10 | 25 | 32 |
| Nonoxynol 9 + FNa (1 mg/l) | 0 | 0 | 0 | 1 | 12 | 20 | 26 | 31 | 32 |
| Nonoxynol 9 + FLi (1 mg/l) | 0 | 0 | 0 | 1 | 16 | 25 | 30 | 32 | – |

Ce tableau illustre l'effet potentialisateur surprenant du fluorure de lithium par rapport au fluorure de sodium. En effet, à concentrations égales, le nombre de souches inhibées en présence de Fli est toujours nettement supérieur au nombre de souches inhibées en présence de FNa. La concentration totalement léthale pour le chlorure de benzalkonium passe de 50 mg/l lorsqu'il est seul, à 25 mg/l en présence de FNa, à 12,5 mg/l en présence de Fli.

Le second essai réalisé a été conduit selon la méthodologie de contact décrite dans la norme AFNOR T 72- 151, en comptant les souches survivantes après un contact substance active/solution microbactérienne de 15 mn suivi d'une filtration sur membrane (0,22 μm de porosité).

Dans cet essai, 26 souches sauvages de Gardnerella Vaginalis ont été testées, en provenance de milieux hospitaliers. On est parti d'une solution à 0,1 % en chlorure de benzalkonium, diluée ensuite à $10^{-1}$, $10^{-2}$, $10^{-3}$ et $10^{-4}$. Les résultats sont résumés dans le tableau ci-après, donnant le nombre total de souches survivantes (sur les 26 testées) en fonction de la dilution.

| Dilution de la solution à 0,1% | Chlorure de benzalkonium seul | Chlorure de benzalkonium f + FNa (1 mg/l) | Chlorure de benzalkonium + FLi (1 mg/l) |
|---|---|---|---|
| $10^{-1}$ | 0 | 0 | 0 |
| $10^{-2}$ | 18 | 7 | 0 |
| $10^{-3}$ | 22 | 15 | 14 |
| $10^{-4}$ | 26 | 26 | 26 |

## EXEMPLE 5 : LUTTE CONTRE LES TRICHOMONAS VAGINALIS

La même méthodologie que dans l'exemple 4 a été utilisée sur 15 souches de Trichomonas Vaginalis. Les résultats sont donnés par le tableau suivant qui fournit le nombre de souches inhibées sur les 15 testées en fonction des concentrations du chlorure de benzalkonium :

| SUBSTANCE | Concentrations en mg/l de chlorure de benzalkonium | | | | | | | | |
|---|---|---|---|---|---|---|---|---|---|
| | 1,56 | 3,125 | 6,25 | 12,5 | 25 | 50 | 100 | 200 | 400 |
| Chlorure de benzalkonium | 1 | 3 | 9 | 13 | 15 | – | – | – | – |
| Chlorure de benzalkonium + Fli (1mg/l) | 3 | 8 | 15 | – | – | – | – | – | – |

La CMI passe donc de 25mg/l pour le chlorure de benzalkonium seul à 6,25mg/l en présence de Fli.

## EXEMPLE 6 :

On a déterminé la CMI du chlorure de benzalkonium, seul et en présence de Fli à 1mg/l sur d'autres souches sauvages en provenance de milieux hospitaliers, à savoir Streptococcus (6 souches), Straphylococcus, Aureus (8 souches), Salmonella Typhimurium (3 souches) et Sarcina Lutea (1 souche).

Les CMI obtenues sont résumées ci-après :

| SOUCHE | Chlorure de benzalkonium seul | Chlorure de benzalkonium en présence de Fli à 1mg/l |
|---|---|---|
| Streptoccocus | 18-20mg/l | 10mg/l |
| Staphylococcus Aureus | 1,56mg/l | 0,90mg/l |
| Salmonella Typhimurium | 17mg/l | 12mg/l |
| Sarcina Lutea | 15mg/l | 11mg/l |

EXEMPLE 7 : LUTTE CONTRE LE BACILLE DE DUCREY

La méthodologie employée est celle décrite par le professeur F. CATALAN, P. SEDNAOUI, A. MILOVANOVIC et Coll. Institut A. FOURNIER, PARIS.

Les essais ont été pratiqués sur 8 souches sauvages d'haemophilus DUCREY. Les résultats suivants ont été enregistrés vis-à-vis de ces souches.

| Principe actif de base | CMI du principe actif de base vis-à-vis du bacille de DUCREY (mg/l) | | |
|---|---|---|---|
| | Principe actif de base seul | en présence de FNa (1 mg/l) | en présence de Fli (1 mg/l) |
| Chlorure de benzalkonium | 100 | 85 | 65 |
| Nonoxynol 9 | 150 | 132,5 | 127,5 |

Le fluorure de lithium s'avère donc être un principe activateur nettement meilleur que le fluorure de sodium.

EXEMPLE 8 : LUTTE CONTRE CANDIDA ALBICANS

Avec la même méthodologie, on a pratiqué des essais sur des souches hospitalières de Candida Albicans.

| Principe actif de base | CMI du principe actif de base | | |
|---|---|---|---|
| | Principe actif de base seul | en présence de FLi à 0,96mg/l | en présence de Fli à 0,96mg/l et de conservateur KATHON CG (marque déposée)à 0,234mg/l |
| Chlorure de benzalkonium | 50mg/l | 36mg/l | 28mg/l |

EXEMPLE 9 : LUTTE CONTRE ASPERGILLUS NIGER

Avec la même méthodologie, les résultats suivants ont été constatés sur des souches hospitalières d'Aspergillus Niger.

| Principe actif de base | CMI du principe actif de base | | |
|---|---|---|---|
| | Principe actif de base seul | en présence de FLi à 0,96mg/l | en présence de Fli à 0,96mg/l et de conservateur KATHON CG (marque déposée)à 0,234mg/l |
| Chlorure de benzalkonium | 150mg/l | 135mg/l | 100mg/l |

EXEMPLE 10 : LUTTE CONTRE LES GERMES PYOGENES

Avec la même méthodologie, les résultats suivants ont été constatés sur des souches hospitalières de germe pyogènes, à savoir Streptocoque β hémolytique, Proteus Vulgaris, Haemophilus Influenzae.

| Principe actif de base | | | CMI du principe actif de base | | |
|---|---|---|---|---|---|
| | | | Streptocoque β hémolytique | Proteus Vulgaris | Haemophilus Influenzae |
| Chlorure de benzalkonium | Seul | | 50mg/l | 25mg/l | 200mg/l |
| | en présence de Fli à 1mg/l et de conservateur KATHON CG ® à 0,4mg/l | | 25mg/l | 18,5mg/l | 150mg/l |

Les formes galéniques préférentielles qui peuvent par exemple être utilisées pour lutter contre les MST sont les mêmes que dans l'application à titre de spermicide.

On peut aussi utiliser le nonoxynol 9 comme principe actif, par exemple :

- <u>OVULE</u> : nonoxynol     0,060mg/l

           Fli           0,01g

Excipients : glycérides semi-synthétiques, conservateur tel que KATHON (marque déposée) q.s.p. 1 ovule.

III- APPLICATION DE L'INVENTION DANS LE DOMAINE DE L'ANTISEPSIE ET DE LA DESINFECTION :

EXEMPLE 11 :

La norme française NF T 72 - 150, Mars 1981 a été suivie pour les essais réalisés. Les essais ont été menés sur les souches définies par la norme AFNOR.

Les résultats obtenus sur les souches définis par la norme AFNOR pour le chlorure de benzalkonium seul et pour le chlorure de benzalkonium avec adjonction de 1mg/l de fluorure de sodium ont déjà été donnés dans la demande de brevet européen EP-A-0 253 037 et ont été enregistrés à nouveau.

Ces résultats sont les suivants :

| SOUCHES | CONCENTRATION MINIMALE INHIBITRICE (CMI) DU CHLORURE DE BENZALKONIUM SEUL | CONCENTRATION MINIMALE INHIBITRICE (CMI) DU CHLORURE DE BENZALKONIUM EN PRESENCE DE FNa A 1mg/l |
|---|---|---|
| Pseudomonas Aeruginosa CNCM A 22 | 31,25 mg/l | 18 mg/l |
| Escherichia Coli CNCM 54 127 | 6,57 mg/l | 3 mg/l |
| Staphylococcus Aureus souche Oxford CNCM 53 154 | 1,56 mg/l | 1,1 mg/l |
| Streptococcus Faecalis CNCM 5 855 | 4 mg/l | 3,6 mg/l |
| Mycobacterium Smegmatis CNCM 7 326 | 30 mg/l | 26 mg/l |

La CMI obtenue pour le chlorure de benzalkonium auquel on a ajouté le fluorure de lithium à 1mg/l est la suivante :

| SOUCHES | CONCENTRATION MINIMALE INHIBITRICE (CMI) DU CHLORURE DE BENZALKONIUM EN PRESENCE DE Fli à 1 mg/l |
|---|---|
| Pseudomonas Aeruginosa CNCM A 22 | 15 mg/litre |
| Escherichia Coli CNCM 54 127 | 2 mg/l |
| Staphylococcus Aureus souche Oxford CNCM 53 154 | 1, 1 mg/l |
| Streptococcus Faecalis CNCM 5 855 | 2, 8 mg/l |
| Mycobacterium Smegmatis CNCM 7 326 | 22 mg/l |

EXEMPLE 12:

Les mêmes essais que dans l'exemple 11 ont été réalisés sur des souches sauvages provenant de milieux hospitaliers à savoir 120 souches de Pseudomonas, 200 souches d'Escherichia Coli, 300 souches de Staphylococcus, 200 souches de Streptococcus et 50 souches de Mycobactérium.

Les résultats obtenus sur les souches sauvages sont résumés dans le tableau suivant :

| SOUCHES | CMI du Chlorure de benzalkonium seul | CMI Chlorure de benzalkonium en présence de FLI à 1 mg/l |
|---|---|---|
| Pseudomonas Aeruginosa | 30 à 120 mg/l | 15 à 80 mg/l |
| Escherichia Coli | 6 à 25 mg/l | 1,8 à 16 mg/l |
| Staphylococcus Aureus | 2 à 13 mg/l | 0,80 à 8,0mg/l |
| Streptococcus Faecalis | 5 à 30 mg/l | 2,5 à 22 mg/l |
| Mycobacterium Smegmatis | 32 à 120 mg/l | 20 à 90 mg/l |

EXEMPLE 13 :

Les mêmes essais que dans l'exemple 12 ont été réalisés sur des souches hospitalières de différents micro-organismes. Les résultats obtenus sont les suivants :

| Souches | CMI du chlorure de benzalkonium seul en mg/l | CMI du chlorure de benzalkonium en mg/l en présence de Fli à 1ppm |
|---|---|---|
| Neisseria Gonorrhocea | 1,15 | 0,60 |
| Trichomonas Vaginalis | 1,30 | 0,90 |
| Candida Albicans | 50 | 35 |
| Gardenerella Vaginalis | 50 | 41 |
| Bacille de Ducrey | 75 | 62 |
| Streptococcus Faecalis | 15 | 9 |
| Staphylococcus Aureus | 1,56 | 1 |
| Aspergillus Niger | 85 | 80 |
| Escherichia Coli | 8 | 6,5 |
| Sarcina Lutea | 11 | 7,5 |
| Bacillus Subtilis | 8 | 5,4 |

EXEMPLE 14 :

Des essais similaires à ceux réalisés aux exemples 12 et 13 ont été réalisés sur 80 souches hospitalières d'Escherichia Coli multirésistant et producteur de β-lactamase plasmidique.

Les résultats sont les suivants :

| Principe actif de base | CMI du principe actif de base en mg/l | | |
|---|---|---|---|
| | Principe actif de base seul | en présence de FLi à 0,96mg/l | en présence de Fli à 0,96mg/l et de conservateur KATHON CG (marque déposée) à 0,47mg/l |
| Chlorure de benzalkonium | 8 à 12 | 6 à 9 | 4 à 6 |

EXEMPLE 15 :

On a comparé l'activité bactéricide de sept antiseptiques désinfectants locaux du commerce avec celle d'une pâte moussante conforme à l'invention contenant :

- chlorure de benzalkonium      2%
- KATHON CG (marque déposée) 0,1%
- fluorure de lithium      0,004%
- fluorure de sodium      1,5%
- excipient : base moussante
et eau purifiée      q.s.p.

La méthodologie employée est celle définie par la norme AFNOR T72-150 en utilisant les produits dans les conditions spécifiées par le fabricant en ce qui concerne les concentrations et les conditions d'utilisation.

Dans le tableau suivant, le signe + indique qu'une croissance des germes a été observée, le signe ++ indique qu'une très forte croissance des germes a été observée et le signe - indique qu'aucune croissance des germes n'a été observée.

Septivon-Lavril est une solution moussante commercialisée par les Laboratoires CLIN MIDY (Paris-France).

Solubacter est une marque déposée désignant une solution moussante commercialisée par le Laboratoire INNOTHERA (Arcueil-France).

Cytéal est une solution moussante commercialisée par les Laboratoires SINBIO (Paris-France).

Hibiscrub est une solution moussante commercialisée par I.C.I.-PHARMA (Cergy-France).

Hibisprint est une solution alcoolique commercialisée par I.C.I.-PHARMA (Cergy-France).

Héxomédine est une marque déposée désignant une solution non moussante commercialisée par THERAPLIX S.A. (France).

Cétavlon est une solution antiseptique commercialisée par I.C.I.-PHARMA (Cergy-France).

Les résultats obtenus sur Escherichia Coli, Staphylococcus Aureus, Streptococcus du groupe A et Streptococcus du Groupe C sont les suivants :

| ANTISEPTIQUES TESTES | Escherichia Coli | Staphylococcus Aureus | Streptococcus du groupe A | Streptococcus du Groupe C |
|---|---|---|---|---|
| Septivon-Lavril | - | ++ | - | - |
| Solubacter (R) | - | - | + | - |
| Cytéal | - | + | + | - |
| Hibiscrub | - | - | - | - |
| Hibisprint | - | ++ | + | + |
| Héxomédine (R) | - | + | - | - |
| Pâte moussante selon l'invention | - | - | - | - |
| Cétavlon | - | - | - | - |

## EXEMPLE 16 :

Des essais similaires à ceux de l'exemple 15, mais mesurant l'activité bactériostatique (capacité à arrêter la prolifération des germes) des antiseptiques ont été réalisés. La pâte moussante selon l'invention était la même que dans l'exemple 15.

Les résultats suivants ont été obtenus (avec les mêmes conventions qu'à l'exemple 15) :

| ANTISEPTIQUES TESTES | Escherichia Coli | Staphylococcus Aureus | Streptococcus du groupe A | Streptococcus du Groupe C |
|---|---|---|---|---|
| Septivon-Lavril | - | + | - | - |
| Solubacter ® | - | - | - | - |
| Cytéal | - | + | + | - |
| Hibiscrub | - | - | - | - |
| Hibisprint | - | + | + | - |
| Héxomédine ® | - | - | - | - |
| Pâte mous- sante selon l'invention | - | - | - | - |
| Cétavlon | - | - | - | - |

EXEMPLE 17 :

La résistance du Pseudomonas Aeruginosa à différents antiseptiques du commerce ou principes actifs réputés bactéricides a été étudiée en fonction de la dureté de l'eau et de la présence ou non de protéines. Le même essai a été réalisé sur la pâte moussante selon l'invention (voir exemple 15).

Mercryl Laurylé est une solution moussante commercialisée par les Laboratoires LABAZ (Paris-France).

Crésol est le nom donné à trois phénols isomères ortho, méta, para $C_7H_8O$ et homologues immédiats du phénol $HO-C_6H_4-CH_3$.

Dans le tableau suivant, R signifie résistant (une pousse de colonies a été observée), S signifie sensible (aucune pousse de notable n'a été observée), P+ signifie que des protéines ont été ajoutées au solvant, et P- signifie que le solvant ne contient pas de protéines.

| PRODUITS ANTISEPTIQUES TESTES | 0,2ml d'innoculum Temps de contact 10mn | | | | 0,4ml d'innoculum Temps de contact 20mn | | | | 0,6ml d'innoculum Temps de contact 30mn | | | |
|---|---|---|---|---|---|---|---|---|---|---|---|---|
| | Eau distillée P+ | P- | Eau dure P+ | P- | Eau distillée P+ | P- | Eau dure P+ | P- | Eau distillée P+ | P- | Eau dure P+ | P- |
| Crésol brut | S | S | S | S | R | S | S | R | R | R | R | R |
| Mercryl Laurylé | R | S | R | R | R | R | R | R | R | R | R | R |
| Solubacter ⓡ | S | S | R | S | R | R | R | R | R | R | R | R |
| Pâte moussante selon l'invention | S | S | S | S | S | S | S | S | S | S | S | S |
| Formaldehyde | S | S | S | S | S | S | S | S | S | S | S | S |
| Héxomédine ⓡ | S | S | S | S | R | S | R | R | R | R | R | R |
| Hibisprint | S | S | S | S | S | S | R | R | R | R | R | R |
| Cétavlon | S | S | R | S | S | S | S | S | S | S | S | S |
| Sulfate de zinc | R | R | R | R | R | R | R | R | R | R | R | R |

Les formes galéniques suivantes peuvent être (par exemple et de façon non limitative) utilisées à titre d'antiseptique local dermatologique (peau, muqueuse, ...) :

- PAIN MOUSSANT :

chlorure de benzalkonium          2%
Fli                               0,04%
Fna                               1,5%
Excipients : base synthétique moussante, conservateur tel que KATHON (marque déposée), eau purifiée, parfum q.s.p. 100%.
- PATE MOUSSANTE : mêmes formulations que pour le pain moussant, sauf pour l'excipient formulé en pâte.

- CREME HYDRATANTE :

| chlorure de benzalkonium | 0,2% |
| Fli | 0,04% |
| Fna | 0,8% |

Excipients : émulgateur, principe hydratant, huile de germe de blé, huile d'amande douce, huile de vaseline, conservateur tel que KATHON (marque déposée), eau purifiée q.s.p. 100%.

IV - APPLICATION DE L'INVENTION DANS LE DOMAINE DE L'ANTIBIOTHERAPIE

EXEMPLE 18 :

L'être vivant unicellulaire choisi pour les essais est le Pseudomonas Aeruginosa (souches sauvages en provenance de milieux hospitaliers).

La même méthodologie que pour les exemples 11 et 12 a été suivie. Les analyses bactériostatiques ont été pratiquées avec un certain nombre d'antibiotiques appartenant aux benzylpénicillines, aminopénicillines et des céphalosporines de première et deuxième génération, à savoir :
– une carboxypénicilline : ticarcilline
– trois uréïdopénicillines : azlocilline, pipéracilline, apalcilline
– cinq céphalosporines : céfopérazone, céfotaxime, ceftriaxone, cefsulodine, ceftazidime.

La détermination de la CMI a été faite par la méthode de microdilution en milieu liquide sur plaques. Les solutions aqueuses de chaque antibiotique sont préparées en bouillon de MUELLER HINTON pour obtenir des dilutions de raison géométrique 2. Le solvant est l'eau bidistillée stérile.

La préparation de l'inoculum bactérien est faite avec une culture de pseudomonas aeruginosa avec agitation au bain-marie à 37°C pendant 4 à 6 heures, donnant $10^6$ bactéries/ml.

On place dans les plaques l'inoculum bactérien et la dilution d'antibiotique, que l'on incube 18 à 24 heures à 37°C -la CMI est lue dans les capsules. Sur 958 souches testées, 235 se sont révélées résistantes à la ticarcilline. Sur ces 235, 69 ne produisent pas de $\beta$-lactamase et 166 produisent une $\beta$-lactamase, à savoir : 50 % une $\beta$-lactamase PSE, 17 % une $\beta$-lactamase TEM, 28 % une $\beta$-lactamase OXA, 5 % une cephalosporinase.

Trois essais ont été conduits par antibiotique et par inoculum bactérien. Les essais ont été conduits d'abord avec les antibiotiques seuls (essais a) ), puis avec ajout à la dilution de chacun d'une dose de 1 mg/l d'une solution aqueuse de fluorure de lithium (essais b) ) dans chaque godet de chaque plaque.

Le tableau suivant synthétise les résultats des essais :

| ESSAIS | ANTI-BIOTIQUE | SOUCHE Tic S βLac⁻ CMI (μg/ml) | SOUCHE Tic R βLac⁻ CMI(μg/ml) | SOUCHE TIC R βLac⁺ CMI (μg/ml) | | | |
|---|---|---|---|---|---|---|---|
| | | | | TEM | PSE | OXA | CEPHALO-SPORINASE |
| 1 | TICARCILLINE | 32 | 512 | 1024 | 4096 | 512 | 256 |
| 2a) | AZLOCILLINE | 8 | 32 | 64 | 64 | 64 | 128 |
| 2b) | + Fli 1mg/l | 4 | 32 | 64 | 64 | 64 | 128 |
| 3a) | PIPERACILLI-NE | 8 | 16 | 64 | 128 | 64 | 128 |
| 3b | + Fli | 8 | 8 | 64 | 64 | 64 | 128 |
| 4a) | APALCILLINE | 4 | 16 | 16 | 64 | 32 | 16 |
| 4b) | + Fli | 2 | 8 | 8 | 32 | 32 | 16 |
| 5a) | CEFOPERAZONE | 8 | 16 | 32 | 64 | 64 | 64 |
| 5b) | + Fli | 4 | 16 | 32 | 32 | 64 | 64 |
| 6a) | CEFOTAXIME | 16 | 32 | 32 | 32 | 32 | 256 |
| 6b) | + Fli | 8 | 16 | 32 | 16 | 16 | 256 |
| 7a) | CEFTRIAXONE | 16 | 32 | 32 | 32 | 32 | 256 |
| 7b) | + Fli | 8 | 16 | 32 | 16 | 32 | ⁓128 |
| 8a) | CEFSULODINE | 4 | 16 | 32 | 32 | 16 | 64 |
| 8b) | + Fli | 4 | 16 | 32 | 16 | 16 | ⁓34 |
| 9a) | CEFTAZIDIME | 4 | 4 | 4 | 2 | 4 | 32 |
| 9b) | + Fli | 2 | 4 | 4 | 2 | 4 | 32 |

Abréviations utilisées :

– Tic S : sensible à la ticarcilline

– Tic R : résistant à la ticarcilline

– βLac⁻ : non productrices de β-Lactamase constitutives

– βLac$^+$ : productices de β-Lactamase constitutives
– CMI : moyenne des CMI

On remarque ainsi que l'ajout de fluorure de lithium (comparaison entre essais a) et essais b) ) permet une diminution de la CMI sur les souches Tic S et Tic R de presque tous les antibiotiques.

EXEMPLE 19 :

Avec la même méthodologie que précédemment, on a déterminé les CMI du chlorure de benzalkonium, seul, en présence du conservateur KATHON CG (marque déposée) (1 mg/l), puis en présence de KATHON CG (marque déposée) (1 mg/l) et de fluorure de lithium (1 mg/l). 200 souches sauvages de pseudomonas aeruginosa ont été testées.

Les résultats suivants ont été observés :

| ANTIBIOTIQUE | SOUCHE TIC S βLac$^-$ | SOUCHE TIC R βLac$^-$ | SOUCHE TIC R βLac$^+$ CMI (mg/l) | | | |
|---|---|---|---|---|---|---|
| | CMI (mg/l) | CMI (mg/l) | TEM | PSE | OXA | CEPHALOSPORINASE |
| TICARCILLINE | 32 | 512 | 1024 | 4096 | 512 | 256 |
| CHLORURE DE BENZALKONIUM | 32 | 64 | 32 | 64 | 64 | 128 |
| + KATHON CG ®  (1 mg/l) | 16 | 32 | 32 | 64 | 32 | 128 |
| + KATHON CG ®  + FLi (1 mg/l) | 16 | 16 | 32 | 16 | 16 | 32 |

On constate donc une forte amélioration en présence du fluorure de lithium, et ce, même sur les souches productrices de céphalosporinase.

EXEMPLE 20 :

Le même essai que dans l'exemple 19 a été conduit, mais avec le nonoxynol 9 comme principe actif de base, 200 souches sauvages de Pseudomonas Aeruginosa ont été aussi testées. Les résultats sont les suivants :

| ANTIBIOTIQUE | SOUCHE TIC S βLac⁻ | SOUCHE TIC R βLac⁻ | SOUCHE TIC R βLac⁺ CMI (mg/l) | | | |
|---|---|---|---|---|---|---|
| | CMI (mg/l) | CMI (mg/l) | TEM | PSE | OXA | CEPHALOSPORINASE |
| TICARCILLINE | 32 | 512 | 1024 | 4096 | 512 | 256 |
| NONOXYNOL 9 | 64 | 128 | 128 | 64 | 128 | 256 |
| + KATHON CG ®  (1 mg/l) | 32 | 128 | 128 | 64 | 128 | 256 |
| + KATHON CG ®  + FLi  (1 mg/l) | 32 | 64 | 64 | 64 | 64 | 128 |

EXEMPLE 21 :

L'activité de la CEFOTAXIME a été testée, seule puis en présence de fluorure de lithium, sur d'autres souches pathogènes que Pseudomonas Aeruginosa.

Le tableau suivant exprime les concentrations (mg/l) de céfotaxime inhibant 50% des souches.

| SOUCHE | CONCENTRATION DE CEFOTAXIME SEULE INHIBITRICE A 50 % | CONCENTRATION DE CEFOTAXIME INHIBITRICE A 50% EN PRESENCE DE FLi à 1 mg/l |
|---|---|---|
| Escherichia Coli K12 J53 | 0.023 | 0.01 |
| Escherichia Coli K12 PIP 111 | 0.023 | 0.023 |
| Escherichia Coli K12 PIP55 | 0.023 | 0.023 |
| Escherichia Coli SOL | 0.18 | 0.18 |
| Klebsiella 1103 | 0.023 | 0.01 |
| Klebsiella U28 | 0.28 | 0.16 |
| Enterobacter T45 | 0.05 | 0.023 |
| Enterobacter P49 | 27.2 | 23.2 |
| Proteus Morganii F20 | 0.01 | 0.01 |
| Serratia 1123 | 0.076 | 0.076 |
| Serratia M01117 | 0.30 | 0.30 |

EXEMPLE 22 :

L'activité antibiotique de l'amoxicilline (pénicilline de type A), seule ou associée au fluorure de lithium a été testée sur des souches hospitalières d'Haemophilus Influenzae productives de β- lactamase.

La méthodologie employée est la même que précédemment.

La concentration d'amoxicilline seule inhibitrice à 100% des souches a varié entre 32ppm et 64ppm.

Une concentration de 6ppm d'amoxicilline en présence de 8ppm de fluorure de lithium a permis d'inhiber à 100% les souches dans un temps variant entre 10 et 12 heures.

A titre de comparaison, une concentration d'amoxicilline de 4 ppm en présence de 1ppm d'acide clavulanique (obtenue par exemple par la spécialité pharmaceutique dénommée AUGMENTIN et commercialisée par les Laboratoires BEE CHAM - SEVIGNE (Paris-France)) a permis d'inhiber à 100% les souches en 24 heures.

On constate donc que l'association de FLi à l'amoxicilline permet de franchir le seuil de sensibilité de 16ppm pour l'Haemophilus Influenzae.

EXEMPLE 23 :

L'activité antiobiotique de l'amoxicilline, seule ou associée au fluorure de lithium a été testée sur des souches hospitalières normalement résistantes à l'amoxicilline (concentration inhibitrice supérieure à 16ppm).
Les résultats sont les suivants :

| SOUCHES | CONCENTRATION INHIBITRICE A 100% D'AMOXICIL-LINE (ppm) | CONCENTRATIONS INHIBITRICES A 100% D'AMOXICILLINE EN PRESENCE DE FLUORURE DE LITHIUM Fli | |
|---|---|---|---|
| | | Amoxicilline (ppm) | Fli (ppm) |
| Staphylococcus Aureus | 32 | 8 | 8 |
| Streptococcus β- hémolytique | 16 | 4 | 8 |
| Klebsiella Pneumoniae | 64 | 2 | 8 |
| Haemophilus Influenzae | > 128 | 32 | 8 |
| Escherichia Coli (plasmide TEM) | > 128 | 32 | 8 |

EXEMPLE 24 :

L'activité antibiotique de l'ampicilline (pénicilline de type A de la famille des bêta-lactamines) seule ou en présence de fluorure de lithium a été testée sur différentes souches.
Les résultats sont les suivants :

| SOUCHES | CONCENTRATION MINIMALE INHIBITRICE DE L'AMPICILLINE EN mg/l | CONCENTRATIONS MINIMALES INHIBITRICES D'AMPICILLINE EN PRESENCE DE FLUORURE DE LITHIUM Fli | |
|---|---|---|---|
| | | Ampicilline en mg/l | Fli en mg/l |
| Escherichia Coli ATCC 25 922 | 4 | 2 | 1 |
| Streptococcus Faecalis ATTC 25 212 | 8 | 4 | 5 |
| Escherichia Coli (10 souches sauvages) | 2-8 | 1-4 | 5 |
| Streptoccocus Faecalis (10 souches sauvages) | 4-8 | 2 | 5 |
| Streptococcus Groupe C | 32 | 8 | 1 |
| Pseudomonas Aeruginosa | 16-32 | 4-8 | 5 |

| SOUCHES RESISTANTES A L'AMPICILLINE | CONCENTRATION MINIMALE INHIBITRICE DE L'AMPICILLINE EN mg/l | CONCENTRATIONS MINIMALES INHIBITRICES D'AMPICILLINE EN PRESENCE DE FLUORURE DE LITHIUM Fli | |
|---|---|---|---|
| | | Ampicilline en mg/l | Fli en mg/l |
| Haemophilus Influenzae (5 souche sauvages) | >128 | 32 | 5 |
| Staphylococcus Aureus (10 souches) | >128 | 64 | 1 |
| Staphylococcus Aureus (souches productrices de bêtalactamase) | >128 | 64 | 6 |
| Enterococcus Faecium | >128 | 64 | 5 |
| Escherichia Coli résistant | 64 | 8 | 5 |

| SOUCHES MULTIRESISTANTES SOUVENT PRODUCTRICES DE β-LACTAMASE | CONCENTRATION MINIMALE INHIBITRICE DE L'AMPICILLINE EN mg/l | CONCENTRATIONS MINIMALES INHIBITRICES D'AMPICILLINE EN PRESENCE DE FLUORURE DE LITHIUM Fli | |
|---|---|---|---|
| | | Ampicilline en mg/l | Fli en mg/l |
| Pseudomonas Aeruginosa | 16-32 | 4-8 | 0,8 |
| Escherichia Coli | 64 | 8 | 0,8 |
| Staphylococcus Aureus | >128 | 32 | 0,8 |
| Streptococcus Groupe C | 256 | 64 | 0,8 |
| Haemophilus Influenzae | >128 | 32 | 0,8 |

EXEMPLE 25 : LUTTE CONTRE LES INFECTIONS BRONCHO-PULMONAIRES

Les mêmes essais que dans les exemples 22 et 23 ont été réalisés avec la tétracycline (famille des cyclines) seule ou en présence de fluorure de lithium sur des souches sauvages hospitalières responsables d'infections broncho-pulmonaires.

| SOUCHES | CONCENTRATION INHIBITRICE A 100% DE TETRACYCLINE (ppm) | CONCENTRATIONS INHIBITRICES A 100% DE TETRACYCLINE EN PRESENCE DE FLUORURE DE LITHIUM Fli | |
|---|---|---|---|
| | | Tétracycline (ppm) | Fli (ppm) |
| Haemophilus Influenzae | 4-8 | 2-4 | 4 |
| Klebsiella Pneumoniae | 0,5 | 0,25 | 2 |

EXEMPLE 26 : LUTTE CONTRE LES INFECTIONS CUTANEES

Les mêmes essais que dans les exemples 22, 23 et 25 ont été réalisés sur des souches sauvages hospitalières responsables d'infections cutanées avec d'une part la polymyxine B seule ou en présence de fluorure de lithium et d'autre part l'érythromycine (famille des macrolides) seule ou en présence de fluorure de lithium.

Les résultats sont les suivants :

| SOUCHES | CONCENTRATION INHIBITRICE A 100% DE POLYMYXINE B (ppm) | CONCENTRATIONS INHIBITRICES A 100% DE POLYMYXINE B EN PRESENCE DE FLUORURE DE LITHIUM FLI | |
|---|---|---|---|
| | | Polymyxine B (ppm) | Fli (ppm) |
| Streptococcus Groupe C | >128 | 64 | 8 |
| Escherichia Coli | >128 | 64 | 2 |
| Staphylococcus Aureus | >128 | 64 | 2 |

| SOUCHES | CONCENTRATION INHIBITRICE A 100% DE D'ERYTHROMYCINE (ppm) | CONCENTRATIONS INHIBITRICES A 100% D'ERYTHROMYCINE EN PRESENCE DE FLUORURE DE LITHIUM Fli | |
|---|---|---|---|
| | | Erythromycine (ppm) | Fli (ppm) |
| Streptococcus Groupe C | 0,25 | 0,125 | 4 |
| Escherichia Coli | 64 | 32 | 8 |

Les différents exemples ci-dessus montrent que l'adjonction du fluorure de lithium avec certains antibiotiques permet d'envisager l'administration orale ou parentérale de ces antibiotiques pour lutter contre des êtres pathogènes que l'on ne savait pas détruire efficacement auparavant.

L'invention concerne donc un produit antibiotique administrable notamment par voie orale, intra-veineuse ou, éventuellement par voie endo-lymphatique, caractérisé en ce qu'il contient d'une part de l'ordre 10mg de fluorure de lithium dans 10 millilitres de soluté injectable, et d'autre part un principe antibiotique, et un produit antibiotique administrable par voie intra-veineuse, caractérisé par des concentrations compatibles avec les caractères physico-chimiques des composants et leur éventuelle toxicité.

**Revendications**

1. Composition spermicide caractérisée en ce qu'elle renferme du lithium ionique ou ionisable, un principe spermicide, notamment un tensioactif surfactif tel qu'un ammonium quaternaire comme le chlorure de benzalkonium, ou un nonoxynol, et un excipient approprié.

2. Composition spermicide caractérisée en ce qu'elle renferme A) du fluorure de lithium, B) un spermicide, notamment un tensioactif surfactif tel qu'un ammonium quaternaire comme le chlorure de benzalkonium, ou un nonoxynol, et C) un excipient approprié.

3. Composition spermicide caractérisée en ce qu'elle renferme A) du fluorure de lithium, B) un spermicide, notamment un tensioactif surfactif tel qu'un ammonium quaternaire comme le chlorure de benzalkonium, ou un nonoxynol, et C) un agent conservateur, notamment comportant un mélange de 5-chloro-2-méthyl-4-isothiazoline-3-one et de 2-méthyl-4-isothiazoline-3-one et D) un excipient approprié.

4. Composition spermicide selon l'une quelconque des revendications 2 et 3, caractérisée en ce qu'elle comporte en outre un autre sel de fluor, notamment du fluorure de sodium.

5. Composition spermicide selon la revendication 2, caractérisée en ce qu'elle contient entre 0,20 % et 0,75 % -notamment de l'ordre de 0,55 %- en poids de fluorure de lithium et entre 0,10 % et 1,20 % en poids de chlorure de benzalkonium.

6. Composition pharmaceutique caractérisée en ce qu'elle comporte du fluorure de lithium et un mélange de 5-chloro-2-méthyl-4-isothiazoline-3-one et de 2-méthyl-4-isothiazoline-3-one.

7. Composition pharmaceutique selon l'une quelconque des revendications 5 et 6 caractérisée en ce qu'elle comporte en outre au moins un principe actif adapté à l'application thérapeutique visée -notamment un tensioactif surfactif tel qu'un ammonium quaternaire comme' le chlorure de benzalkonium, ou un nonoxynol, ou un antibiotique, ou un bactéricide, ou un sporicide, ou un fongicide, ou un virucide-.

8. Composition pharmaceutique caractérisée en ce qu'elle comporte du lithium ionique ou ionisable et un principe antibiotique.

9. Composition pharmaceutique selon la revendication 8 caractérisée en ce qu'elle comporte du fluorure de lithium et un principe antibiotique.

10. Composition destinée à la désinfection de surfaces telles que les sols ou les instruments caractérisée en ce qu'elle renferme du lithium ionique et ionisable, un principe désinfectant et un excipient approprié.

11. Composition destinée à la désinfection de surfaces telles que les sols ou les instruments caractérisée en ce qu'elle renferme A) du fluorure de lithium, B) un tensioactif surfactif tel qu'un ammonium quaternaire comme le chlorure de benzalkonium, ou un nonoxynol, et C) un excipient approprié.

12. Composition selon la revendication 11 caractérisée en ce qu'elle renferme en outre un agent conservateur, notamment comportant un mélange de 5-chloro-2-méthyl-4-isothiazoline-3-one et de 2-méthyl-4-isothiazoline-3-one.

13. Composition selon l'une quelconque des revendications 11 et 12 caractérisée en ce qu'elle comporte en outre un autre sel de fluor, notamment du fluorure de sodium.

14. Application du cation $Li^+$ lithium pour obtenir une composition destinée à détruire ou inhiber les gamètes - notamment les spermatozoïdes- à titre de contraceptif local.

15. Application du cation $Li^+$ lithium pour obtenir une composition pharmaceutique destinée à détruire ou inhiber un être vivant unicellulaire ou virus en dehors de la sphère bucco-dentaire

16. Application du cation $Li^+$ lithium pour obtenir une composition destinée à lutter contre les maladies sexuellement transmissibles.

17. Application du cation $Li^+$ lithium pour obtenir une composition antibiotique.

18. Application du cation $Li^+$ lithium pour obtenir une composition antiseptique pour la désinfection locale du corps humain ou animal en dehors de la sphère bucco-dentaire.

19. Application d'au moins un composé susceptible de libérer l'ion $F^-$ et l'ion $Li^+$ -notamment du fluorure de lithium- pour obtenir une composition pharmaceutique destinée à détruire ou inhiber un être vivant unicellulaire ou virus en dehors de la sphère bucco-dentaire.

20. Application d'au moins un composé susceptible de libérer l'ion $F^-$ et l'ion $Li^+$ -notamment du fluorure de lithium- pour obtenir une composition destinée à lutter contre les maladies sexuellement transmissibles.

21. Application d'au moins un composé susceptible de libérer l'ion $F^-$ et l'ion $Li^+$ -notamment du fluorure de lithium- pour obtenir une composition contraceptive locale.

22. Application d'au moins un composé susceptible de libérer l'ion $F^-$ et l'ion $Li^+$ -notamment du fluorure de lithium- pour obtenir une composition antibiotique.

30

23. Application d'au moins un composé susceptible de libérer l'ion F⁻ et l'ion Li⁺ -notamment du fluorure de lithium- pour obtenir une composition antiseptique pour la désinfection locale du corps humain ou animal en dehors de la sphère bucco-dentaire.

24. Application d'au moins un composé susceptible de libérer l'ion F⁻, d'au moins un composé susceptible de libérer l'ion Li⁺ -notamment du fluorure de lithium- et d'au moins un principe actif -notamment un tensioactif surfactif tel qu'un ammonium quaternaire comme le chlorure de benzalkonium, ou un nonoxynol- pour obtenir une composition pharmaceutique destinée à détruire ou inhiber un virus ou un être vivant unicellulaire en dehors de la sphère bucco-dentaire.

25. Application d'au moins un composé susceptible de libérer l'ion F⁻, d'au moins un composé susceptible de libérer l'ion Li⁺ -notamment du fluorure de lithium- et d'au moins un principe actif -notamment un tensioactif surfactif tel qu'un ammonium quaternaire comme le chlorure de benzalkonium, ou un nonoxynol- pour obtenir une composition contraceptive locale.

26. Application d'au moins un composé susceptible de libérer l'ion F⁻, d'au moins un composé susceptible de libérer l'ion Li⁺ -notamment du fluorure de lithium- et d'au moins un principe actif -notamment un tensioactif surfactif tel qu'un ammonium quaternaire comme le chlorure de benzalkonium, ou un nonoxynol- pour obtenir une composition antibiotique.

27. Application d'au moins un composé susceptible de libérer l'ion F⁻, d'au moins un composé susceptible de libérer l'ion Li⁺ -notamment du fluorure de lithium- et d'au moins un principe actif -notamment un tensioactif surfactif tel qu'un ammonium quaternaire comme le chlorure de benzalkonium, ou un nonoxynol- pour obtenir une composition destinée à lutter contre les maladies sexuellement transmissibles.

28. Application d'au moins un composé susceptible de libérer l'ion F⁻, d'au moins un composé susceptible de libérer l'ion Li⁺ -notamment du fluorure de lithium- et d'au moins un principe actif -notamment un tensioactif surfactif tel qu'un ammonium quaternaire comme le chlorure de benzalkonium, ou un nonoxynol- pour obtenir une composition antiseptique pour la désinfection locale du corps humain ou animal en dehors de la sphère bucco-dentaire.

29. Application d'au moins un composé susceptible de libérer l'ion F⁻, d'au moins un composé susceptible de libérer l'ion Li⁺ -notamment du fluorure de lithium- et d'au moins un principe antibiotique pour obtenir un produit antibiotique comportant des concentrations en F⁻, Li⁺ et en principe antibiotique suffisamment faibles pour autoriser son administration par voie orale ou parentérale.

30. Application d'au moins un composé susceptible de libérer l'ion F⁻, d'au moins un composé susceptible de libérer l'ion Li⁺ -notamment du fluorure de lithium- et d'au moins un principe virucide -notamment un tensioactif surfactif tel qu'un ammonium quaternaire comme le chlorure de benzalkonium, ou un nonoxynol- pour obtenir un produit virucide comportant des concentrations en F⁻, Li⁺ et en principe virucide de base suffisamment faibles pour autoriser son administration par voie orale ou parentérale.

31. Application d'au moins un composé susceptible de libérer l'ion F⁻, d'au moins un composé susceptible de libérer l'ion Li⁺ -notamment du fluorure de lithium- et d'au moins un principe bactéricide ou antibiotique ou fongicide, pour obtenir une composition destinée à lutter contre les micro-organismes pathogènes tels que Chlamydiae, Gardnerella Vaginalis, Bacille de Ducrey, Candida Albicans, Aspergillus, Streptotoccus, Proteus Vulgaris, Haemophilus Influenzae, Pseudomonas Aeruginosa, Escherichia Coli, Staphylococcus, Mycobacterium, Neisseria Gonorrhoaea, Trichomonas, Treponomas, Sarcina Lutea, Bacillus Subtilis, Klebsiella Pneumoniae, Enterococcus.

32. Application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition pharmaceutique destinée à détruire ou inhiber un virus ou un être vivant unicellulaire.

33. Application selon l'une quelconque des revendications 25 à 27 A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition contraceptive locale, ou une composition antibiotique, ou une composition destinée à lutter contre les maladies sexuellement transmissibles.

34. Application A) du fluorure de lithium, B) d'un tensioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, et D) d'un excipient approprié, pour obtenir une composition antiseptique pour la désinfection locale du corps humain ou animal.

35. Application selon l'une quelconque des revendications 32 à 34 A) du fluorure de lithium, B) d'un ten-

EP 0 310 476 B1

sioactif surfactif tel qu'un ammonium quaternaire comme le chlorure de benzalkonium, ou un nonoxynol, C) d'un agent conservateur, notamment comportant un mélange de 5-chloro-2-méthyl-4-isothiazoline-3-one et de 2-méthyl-4-isothiazoline-3-one, D) d'un excipient approprié, et E) d'un autre sel de fluor, notamment du fluorure de sodium, pour obtenir une composition pharmaceutique destinée à détruire ou inhiber un virus ou un être vivant unicellulaire, ou une composition contraceptive locale, ou une composition antibiotique, ou une composition destinée à lutter contre les maladies sexuellement transmissibles, ou une composition antiseptique pour la désinfection locale du corps humain ou animal.

**Patentansprüche**

1. Spermizide Zusammensetzung, **dadurch gekennzeichnet**, daß sie ionisches oder ionisierbares Lithium, einen spermiziden Werkstoff, insbesondere einen grenzflächenaktiven Stoff, z.B. eine quartäre Ammoniumverbindung wie Benzalkoniumchlorid oder ein Nonoxinol, und eine geeignete Grundmasse enthält.

2. Spermizider Zusammensetzung, dadurch gekennzeichnet, daß sie A) Lithiumfluorid, B) ein Spermizid, insbesondere einen grenzflächenaktiven Stoff, z.B. eine quartäre Ammoniumverbindung wie Benzalkoniumchlorid oder ein Nonoxinol, und C) eine geeignete Grundmasse enthält.

3. Spermizide Zusammensetzung, dadurch gekennzeichnet, daß sie A) Lithiumfluorid, B) ein Spermizid, insbesondere einen grenzflächenaktiven Stoff, z.B. eine quartäre Ammoniumverbindung wie Benzalkoniumchlorid oder ein Nonoxinol, C) ein Konservierungsmittel, insbesondere aus einer Mischung von 5-Chlor-2-Methyl-4-Isothiazolin-3-On und 2-Methyl-4-Isothiazolin-3-On, und D) eine geeignete Grundmasse enthält.

4. Spermizide Zusammensetzung nach Anspruch 2 oder 3, dadurch gekennzeichnet, daß sie außerdem ein anderes Fluorsalz, insbesondere Natriumfluorid enthält.

5. Spermizide Zusammensetzung nach Anspruch 2, dadurch gekennzeichnet, daß sie zwischen 0,20 und 0,75 Gew.-% - insbesondere in der Größenordnung von 0,55 Gew.-% - Lithiumfluorid und zwischen 0,10 und 1,20 Gew.-% Benzalkoniumchlorid enthält.

6. Pharmazeutische Zusammensetzung, dadurch gekennzeichnet, daß sie Lithiumfluorid und eine Mischung aus 5-Chlor-2-Methyl-4-Isothiazolin-3-On und 2-Methyl-4-Isothiazolin-3-On enthält.

7. Pharmazeutische Zusammensetzung nach Anspruch 5 oder 6, dadurch gekennzeichnet, daß sie außerdem mindestens einen an die gezielte therapeutische Anwendung angepaßten Wirkstoff - insbesondere eine grenzflächenaktiven Stoff, z.B. eine quartäre Ammoniumverbindung wie Benzalkoniumchlorid oder ein Nonoxinol oder ein Antibiotikum oder ein Bakterizid oder ein Sporizid oder ein Fungizid oder ein Viruzid - enthält.

8. Pharmazeutische Zusammensetzung, dadurch gekennzeichnet, daß sie ionisches oder ionisierbares Lithium und einen antibiotischen Wirkstoff enthält.

9. Pharmazeutische Zusammensetzung nach Anspruch 8, dadurch gekennzeichnet, daß sie Lithiumfluorid und einen antibiotischen Wirkstoff enthält.

10. Zusammensetzung zur Desinfektion von Oberflächen, wie z.B. Böden oder Instrumente, dadurch gekennzeichnet, daß sie ionisches oder ionisierbares Lithium, einen desinfizierenden Wirkstoff und eine geeignete Grundmasse enthält.

11. Zusammensetzung zur Desinfektion von Oberflächen, wie z.B. Böden oder Instrumente, dadurch gekennzeichnet, daß sie A) Lithiumfluorid, B) einen grenzflächenaktiven Stoff, z.B. eine quartäre Ammoniumverbindung, Benzalkoniumchlorid oder ein Nonoxinol, und C) eine geeignete Grundmasse enthält.

12. Zusammensetzung nach Anspruch 11, dadurch gekennzeichnet, daß sie außerdem ein Konservierungsmittel, insbesondere aus einer Mischung von 5-Chlor-2-Methyl-4-Isothiazolin-3-On und 2-Methyl-4-Isothiazolin-3-On enthält.

13. Zusammensetzung nach Anspruch 11 oder 12, dadurch gekennzeichnet, daß sie außerdem ein anderes Fluorsalz, insbesondere Natriumfluorid, enthält.

14. Anwendung des Lithiumkations Li$^+$, um eine Zusammensetzung zur Zerstörung oder Hemmung von Gameten - insbesondere von Spermatozoen - als lokales Kontrazeptivum zu erhalten.

15. Anwendung des Lithiumkations Li$^+$, um eine pharmazeutische Zusammensetzung zur Zerstörung oder Hemmung einzelliger Lebewesen oder Viren außerhalb des bucco-dentalen Bereichs zu erthalten.

16. Anwendung des Lithiumkations Li$^+$, um eine Zusammensetzung zur Bekämpfung sexuell übertragbarer Krankheiten zu erhalten.

17. Anwendung des Lithiumkations Li$^+$, um eine antibiotische Zusammensetzung zu erhalten.

18. Anwendung des Lithiumkations Li$^+$, um eine antiseptische Zusammensetzung zur lokalen Desinfektion des menschlichen oder tierischen Körpers außerhalb des bucco-dentalen Bereichs zu erhalten.

19. Anwendung mindestens einer zur Freisetzung des Ions F$^-$ und des Ions L+ - insbesondere aus Lithiumfluorid - geeigneten Verbindung, um eine pharmazeutische Zusammensetzung zur Zerstörung oder Hemmung einzelliger Lebewesen oder Viren außerhalb des bucco-dentalen Bereichs zu erhalten.

20. Anwendung mindestens einer zur Freisetzung des Ions F⁻ und des Ions Li⁺ - insbesondere aus Lithiumfluorid - geeigneten Verbindung, um eine Zusammensetzung zur Bekämpfung sexuell übertragbarer Krankheiten zu erhalten.

21. Anwendung mindestens einer zur Freisetzung des Ions F⁻ und des Ions Li⁺ - insbesondere aus Lithiumfluoird - geeigneten Verbindung, um eine lokal kontrazeptive Zusammensetzung zu erhalten.

22. Anwendung mindestens einer zur Freisetzung des Ions F⁻ und des Ions Li⁺ - insbesondere aus Lithiumfluorid - geeigneten Verbindung, um eine antibiotische Zusammensetzung zu erhalten.

23. Anwendung mindestens einer zur Freisetzung des Ions F⁻ und des Ions Li⁺ - insbesondere aus Lithiumfluorid - geeigneten Verbindung, um eine antiseptische Zusammensetzung zur lokalen Desinfektion des menschlichen oder tierischen Körpers außerhalb des bucco-dentalen Bereichs zu erhalten.

24. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines Wirkstoffs - insbesondere eines grenzflächenaktiven Stoffs, z.B. einer quartären Ammoniumverbindung, wie Benzalkoniumchlorid oder eines Nonoxinols -, um eine pharmazeutische Zusammensetzung zur Zerstörung oder Hemmung von Viren oder einzelligen Lebewesen außerhalb des bucco-dentalen Bereichs zu erhalten.

25. Anwendung mindestens einer zur Freisetzung des Ions F⁻ geeigneten Verbindung, mindestens einer zur Freisetzung des Ions Li⁺ geeigneten Verbindung - insebesondere aus Lithiumfluorid - und mindestens eines Wirkstoffs - insbesondere eines grenzflächenaktiven Stoffs, z.B. einer quartären Ammoniumverbindung, wie Benzalkoniumchlorid oder eines Nonoxinols -, um eine lokal kontrazeptive Zusammensetzung zu erhalten.

26. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines Wirkstoffs - insbesondere eines grenzflächenaktiven Stoffs, z.B. einer quartären Ammoniumverbindung, wie Benzalkoniumchlorid oder eines Nonoxinols -, um eine antibiotische Zusammensetzung zu erthalten.

27. Anwzendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines Wirkstoffs - insbesondere eines grenzflächenaktiven Stoffs, z.B. einer quartären Ammoniumverbindung, wie Benzalkoniumchlorid oder eines Nonoxinols -, um eine Zusammensetzung zur Bekämpfung sexuell übertragbarer Krankheiten zu erhalten.

28. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines Wirkstoffs - insbesondere eines grenzflächenaktiven Stoffs, z.B. einer quartären Ammoniumverbindung wie Benzalkoniumchlorid oder eines Nonoxinols -, um eine antiseptische Zusammensetzung zur lokalen Desinfektion des menschlichen oder tierischen Körpers außerhalb des bucco-dentalen Bereichs zu erhalten.

29. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines antibiotischen Wirkstoffs, um ein antibiotisches Produkt zu erhalten, das genügend geringe Konzentrationen an F⁻, Li⁺ und antibiotischem Wirkstoff enthält, um seine orale oder parentale Verabreichung zu erlauben.

30. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines viruziden Wirkstoffs - insbesondere eines grenzflächenaktiven Stoffes, z.B. einer quartären Ammoniumverbindung wie Benzalkoniumchlorid oder eines Nonoxinols -, um ein viruzides Produkt zu erhalten, das genügend geringe Konzentrationen an F⁻, Li⁺ und viruzidem Wirkstoff enthält, um seine orale oder parenterale Verabreichung zu erlauben.

31. Anwendung mindestens einer zur Freisetzung des Ions F⁻ geeigneten Verbindung, mindestens einer zur Freisetzung des Ions Li⁺ geeigneten Verbindung - insbesondere aus Lithiumfluorid - und mindestens eines bakteriziden, antibiotischen oder fungiziden Wirkstoffs, um eine Zusammensetzung zur Bekämpfung pathogener Mikroorganismen, wie z.B. Chlamydiae, Gardnerella Vaginalis, Ducrey-Bazillus, Candida Albicans, Aspergillus, Streptotoccus, Proteus Vulgaris, Haemophilus Influenzae, Pseudomonas Aeruginosa, Escherichia Coli, Staphylococcus, Mycobacterium, Neisseria Gonorrhoaca, Trichomonas, Treponomas, Sarcina Lutea, Bacillus Subtilis, Klebsiella Pneumoniae und Enterococcus zu erhalten.

32. Anwendung A) von Lithiumfluorid, B) eines grenzflächenaktiven Stoffs, z.B. einer quartären Ammoniumverbindung wie Benzalkoniumchlorid oder eines Nonoxinols, C) eines Konservierungsmittels, insbesondere aus einer Mischung von 5-Chloro-2-Methyl-4-Isothiazolin-3-On und 2-Methyl-4-Isothiazolin-3-On, und D) einer geeigneten Grundmasse, um eine pharmazeutische Zusammensetzung zur Zerstörung oder Hemmung von Viren oder einzelligen Lebewesen zu erhalten.

33. Anwendung nach einem der Ansprüche 25 bis 27 A) von Lithiumfluorid, B) eines grenzflächenaktiven Stoffs, z.B. einer quartären Ammoniumverbindung wie Benzalkoniumchlorid oder eines Nonoxinols, C) eines Konservierungsmittels, insbesondere aus einer Mischung von 5-Chlor-2-Methyl-4-Isothiazolin-3-On und 2-Methyl-4-Isothiazolin-3-On, und D) einer geeigneten Grundmasse, um eine lokal kontrazeptive Zusammensetzung oder eine antibiotische Zusammensetzung oder eine Zusammensetzung zur Bekämpfung sexuell übertragbarer Krankheiten zu erhalten.

34. Anwendung A) von Lithiumfluorid, B) eines grenzflächenaktiven Stoffs, z.B. einer quartären Ammoniumverbindung wie Benzalkoniumchlorid oder eines Nonoxinols, C) eines Konservierungsmittels, insbesondere aus einer Mischung von 5-Chloro-2-Methyl-4-Isothiazolin-3-On und 2-Mehtyl-4-Isothiazolin-3-On, und D) einer geeigneten Grundmasse, um eine antiseptische Zusammensetzung zur lokalen Desinfektion des menschlichen oder tierischen Körpers zu erhalten.

35. Anwendung nach einem der Ansprüche 32 bis 34 A) von Lithiumfluorid, B) eines grenzflächenaktiven Stoffs, z.B. einer quartären Ammoniumverbindung, wie Benzalkoniumchlorid oder eines Nonoxinols, C) eines Konsiervierungsmittels, insbesondere aus einer Mischung von 5-Chlor-2-Methyl-4-Isothiazolin-3-On und 2-Methyl-4-Isothiazolin-3-On, D) einer geeigneten Grundmasse, und E) eines anderen Fluorsalzes, insbesondere Natriumfluorid, um eine pharmazeutische Zusammensetzung zur Zerstörung oder Hemmung von Viren oder einzelligen Lebewesen oder eine lokalkontrazeptive Zusammensetzung oder eine antibiotische Zusammensetzung oder eine Zusammensetzung zur Bekämpfung sexuell übertragbarer Krankheiten oder eine antiseptische Zusammensetzung zur lokalen Desinfektion des menschlichen oder tierischen Körpers zu erhalten.

## Claims

1. A spermicidal composition characterized in that it includes ionised or ionisable lithium, a spermicidal ingredient, in particular a surface-active agent such as a quaternary ammonium salt like benzalkonium chloride, or a nonoxynol, and an appropriate excipient.

2. A spermicidal composition characterized in that it includes (A) lithium fluoride, (B) a spermicide, in particular a surface-active agent such as a quaternary ammonium salt like benzalkonium chloride, or a nonoxynol, and (C) an appropriate excipient.

3. A spermicidal composition characterized in that it includes (A) lithium fluoride, (B) a spermicide, in particular a surface-active agent such as a quaternary ammonium salt like benzalkonium chloride, or a nonoxynol, and (C) a preserving agent, in particular comprising a mixture of 5-chloro-2-methyl-4-isothiazoline-3-one and 2-methyl-4-isothiazoline-3-one, and (D) an appropriate excipient.

4. A spermicidal composition according to claim 2 or 3, characterized in that it also includes another fluorine salt, in particular sodium fluoride.

5. A spermicidal composition according to claim 2, characterized in that it contains 0.20% to 0.75% by weight - in particular about 0.55% - of lithium fluoride and 0.10% to 1.20% by weight of benzalkonium chloride.

6. A pharmaceutical composition characterized in that it includes lithium fluoride and a mixture of 5-chloro-2-methyl-4-isothiazoline-3-one and 2-methyl-4-isothiazoline-3-one.

7. A pharmaceutical composition according to claim 5 or 6 characterized in that it also includes at least one active ingredient adapted to the intended therapeutic use - in particular a surface-active agent such as a quaternary ammonium salt like benzalkonium chloride, or a nonoxynol, or an antibiotic, or a bactericide or a sporicide or a fungicide or a viricide.

8. A pharmaceutical composition characterised in that it includes ionised or ionisable lithium and an antibiotic ingredient.

9. A pharmaceutical composition according to claim 8 characterized in that it includes lithium fluoride and an antibiotic ingredient.

10. A composition for disinfecting surfaces such as floors or instruments characterized in that it includes ionised or ionisable lithium, a disinfectant ingredient and an appropriate excipient.

11. A composition for disinfecting surfaces such as floors or instruments characterized in that it includes (A) lithium fluoride, (B) a surface-active agent such as quaternary ammonium salt like benzalkonium chloride, or a nonoxynol, and (C) an appropriate excipient.

12. A composition according to claim 11, characterized in that it also includes a preserving agent, in particular comprising a mixture of 5-chloro-2-methyl-4-isothiazoline-3-one and 2-methyl-4-isothiazoline-3-one.

13. A composition according to claim 11 or 12 characterized in that it also includes another fluorine salt, in particular sodium fluoride.

14. The use of the $Li^+$ cation to obtain a composition for destroying or inhibiting gametes - in particular spermatozoa - as a local contraceptive.

15. The use of the $Li^+$ cation to obtain a pharmaceutical composition for destroying or inhibiting a unicellular organism or virus outside the bucco-dental area.

16. The use of the Li$^+$ cation to obtain a composition for combating sexually transmitted diseases.

17. The use of the Li$^+$ cation to obtain an antibiotic composition.

18. The use of the Li$^+$ cation to obtain an antiseptic composition for local disinfection of the human or animal body outside the bucco-dental area.

19. The use of at least one compound capable of liberating the F$^-$ ion and the Li$^+$ ion - in particular from lithium fluoride - to obtain a pharmaceutical composition for destroying or inhibiting a unicellular organism or virus outside the bucco-dental area.

20. The use of at least one compound capable of liberating the F$^-$ ion and the Li$^+$ ion - in particular from lithium fluoride - to obtain a composition for combating sexually transmitted diseases.

21. The use of at least one compound capable of liberating the F$^-$ ion and the Li$^+$ ion - in particular from lithium fluoride - to obtain a local contraceptive composition.

22. The use of at least one compound capable of liberating the F$^-$ ion and the Li$^+$ ion - in particular from lithium fluoride - to obtain an antibiotic composition.

23. The use of at least one compound capable of liberating the F$^-$ ion and the Li$^+$ ion - in particular from lithium fluoride - to obtain an antiseptic composition for local disinfection of the human or animal body outside the bucco-dental area.

24. The use of at least one compound capable of liberating the F$^-$ ion and the Li$^+$ ion - in particular from lithium fluoride - and of at least one active ingredient - in particular a surface-active agent such as a quaternary ammonium salt like benzalkonium chloride, or a nonoxynol - to obtain a pharmaceutical composition for destroying or inhibiting a virus or a unicellular organism outside the bucco-dental area.

25. The use of at least one compound capable of liberating the F$^-$ ion, of at least one compound capable of liberating the Li$^+$ ion - in particular from lithium fluoride - and of at least one active ingredient - in particular a surface-active agent such as a quaternary ammonium salt like benzalkonium chloride, or a nonoxynol - to obtain a local contraceptive composition.

26. The use of at least one compound capable of liberating the F$^-$ ion, of at least one compound capable of liberating the Li$^+$ ion - in particular from lithium fluoride - and of at least one active ingredient - in particular a surface-active agent such as a quaternary ammonium salt like benzalkonium chloride, or a nonoxynol - to obtain an antibiotic composition.

27. The use of at least one compound capable of liberating the F$^-$ ion, of at least one compound capable of liberating the Li$^+$ ion - in particular from lithium fluoride - and of at least one active ingredient - in particular a surface-active agent such as a quaternary ammonium salt like benzalkonium chloride, or a nonoxynol - to obtain a composition for combating sexually transmitted diseases.

28. The use of at least one compound capable of liberating the F$^-$ ion, of at least one compound capable of liberating the Li$^+$ ion - in particular from lithium fluoride - and of at least one active ingredient - in particular a surface-active agent such as a quaternary ammonium salt like benzalkonium chloride, or a nonoxynol - to obtain an antiseptic composition for local disinfection of the human or animal body outside the bucco-dental area.

29. The use of at least one compound capable of liberating the F$^-$ ion, of at least one compound capable of liberating the Li$^+$ ion - in particular from lithium fluoride - and of at least one active antibiotic ingredient to obtain an antibiotic product containing sufficiently low concentrations of F$^-$, Li$^+$ and an antibiotic ingredient to allow oral or parenteral administration.

30. The use of at least one compound capable of liberating the F$^-$ ion, of at least one compound capable of liberating the Li$^+$ ion - in particular from lithium fluoride - and of at least one viricidal ingredient - in particular a surface-active agent such as a quaternary ammonium salt like benzalkonium chloride, or a nonoxynol - to obtain a viricidal product containing sufficiently low concentrations of F$^-$, Li$^+$ and a basic viricidal ingredient to allow oral or parenteral administration.

31. The use of at least one compound capable of liberating the F$^-$ ion, of at least one compound capable of liberating the Li$^+$ ion - in particular from lithium fluoride - and of at least one bactericidal or antibiotic or fungicidal ingredient to obtain a composition for combating pathogenic microorganisms such as Chlamydiae, Gardnerella Vaginalis, Bacillus Ducrey, Candida Albicans, Aspergillus, Streptococcus, Proteus Vulgaris, Haemophilus Influenzae, Pseudonomas Aeruginosa, Escherichia Coli, Staphylococcus, Mycobacterium, Neisseria Gonorrhoea, Trichomonas, Treponomas, Sarcina Lutea, Bacillus Subtilis, Klebsiella Pneumoniae and Enterococcus.

32. The use of (A) lithium fluoride, (B) a surface-active agent such as a quaternary ammonium salt like benzalkonium chloride, or a nonoxynol, (C) a preserving agent, in particular including a mixture of 5-chloro-2-methyl-4-isothiazoline-3-one and 2-methyl-4-isothiazoline-3-one, and (D) an appropriate excipient, to obtain a pharmaceutical composition for destroying or inhibiting a virus or unicellular organism.

33. The use according to any of claims 25 to 27 of (A) lithium fluoride, (B) a surface-active agent such as a quaternary ammonium salt like benzalkonium chloride, or a nonoxynol, (C) a preserving agent, in particular including a mixture of 5-chloro-2-methyl-4-isothiazoline-3-one and 2-methyl-4-isothiazoline-3-one, and (D) an appropriate excipient, to obtain a local contraceptive composition, or an antibiotic composition, or a composition for combating sexually transmitted diseases.

34. The use of (A) lithium fluoride, (B) a surface-active agent such as a quaternary ammonium salt like benzalkonium chloride, or a nonoxynol, (C) a preserving agent, in particular comprising a mixture of 5-chloro-2-methyl-4-isothiazoline-3-one and 2-methyl-4-isothiazoline-3-one, and (D) an appropriate excipient, to obtain an antiseptic composition for local disinfection of the human or animal body.

35. The use according to any of claims 32 to 34 of (A) lithium fluoride, (B) a surface-active agent such as a quaternary ammonium salt like benzalkonium chloride, or a nonoxynol, (C) a preserving agent, in particular comprising a mixture of 5-chloro-2-methyl-4-isothiazoline-3-one and 2-methyl-4-isothiazoline-3-one, (D) an appropriate excipient, and (E) another fluorine salt, in particular sodium fluoride, to obtain a pharmaceutical composition, for destroying or inhibiting a virus or a unicellular organism, or a local contraceptive composition, or an antibiotic composition, or a composition for combating sexually transmitted diseases, or an antiseptic composition for local disinfection of the human or animal body.